# EUROPÄISCHE PATENTSCHRIFT

(11) **EP 2 459 124 B1**
(45) Veröffentlichungstag und Bekanntmachung des Hinweises auf die Patenterteilung: **20.04.2016**
(21) Anmeldenummer: 10751780.7
(22) Anmeldetag: 28.07.2010
(51) Int. Cl.: A61F 2/30, A61F 2/36

(54) **GELENKPROTHESEN-SYSTEM**
JOINT PROSTHESIS SYSTEM
SYSTÈME DE PROTHÈSE D'ARTICULATION

(30) Priorität: 29.07.2009 DE 102009035259
(43) Veröffentlichungstag der Anmeldung: 06.06.2012
(73) Patentinhaber: Merete Medical GmbH, 12247 Berlin (DE)
(72) Erfinder: KRANZ, Curt, 14163 Berlin (DE); ANAPLIOTIS, Emmanuel, 14195 Berlin (DE); HILSE, Martin, 12105 Berlin (DE)
(74) Vertreter: Hannig, Wolf-Dieter
(86) Internationale Anmeldenummer: PCT/DE2010/000881
(87) Internationale Veröffentlichungsnummer: WO 2011/012115

(56) Entgegenhaltungen:
- EP-A1- 0 385 572
- EP-A2- 0 562 782
- DE-A1- 19 904 437
- US-A- 5 066 304
- US-A1- 2006 188 845

## Beschreibung

Die Erfindung betrifft ein Gelenkprothesen-System mit einem in den Femur implantierten metallischen Schaft mit einem einen Außenkonus aufweisenden Hals, einer Gelenkkugel aus Keramik mit einer einen Innenkonus umfassenden Aufnahme für den Hals und einem hülsenförmigen Adapter, der zwischen Schaft und Gelenkkugel klemmend angeordnet ist, wobei durch eine besondere Elastizität und spezielle Formgebung eines inneren und äußeren Wandabschnittes des Adapters die Krafteinleitung auf einen Schmalen Flächenbereich am metallischen Außenkonus und die Krafteinleitung in den keramischen Innenkonus flächig auf einen dem schmalen Flächenbereich gegenüberliegenden lasttragenden Bereich innerhalb der größten Masseausdehnung der keramischen Gelenkkugel konzentriert ist.

### Stand der Technik

Aus der DE 199 04 437 A1 ist eine Klemmsitz-Verbindung zwischen Prothesenkomponenten einer GelenkProthese bekannt, bei der die eine Prothesenkomponente in das Knochengewebe eingesetzt ist und einen Konus trägt, auf dem ein Kugelkopf aufgesetzt ist, der einen anderen, pfannenförmigen Gelenkpartner artikuliert, wobei zwischen dem Konus und dem Kugelkopf ein Kopplungselement zur Homogenisierung der Kraftübertragung angeordnet ist. Die Elastizität und die Dämpfungseigenschaften dieses Kopplungselementes sind durch seine Porosität und die Struktur seiner Oberfläche vorgebbar. Das Kopplungselement kann aus einem Wickelkörper, Sinterkörper oder Schwammkörper aus biokompatiblem Material bestehen.
Dieser Stand der Technik steht stellvertretend für weitere bekannte Lösungen wie beispielsweise EP 385 572 B1, DE 91 03 574 U1, DE 195 17 843 A1, DE 196 40 745 A1, EP 1 124 507 A1, US 5 066 304A1, DE 199 04 437A1, US 2006/0188845A1, EP 562 782A1.

Bei all diesen bekannten Lösungen kommt das Kopplungselement zwischen Schaft und Gelenkkugel vollkommen flächig zur Anlage, so dass die Krafteinleitung bei der Bewegung, die im Einzelfall ohne Weiteres das Fünffache des Körpergewichtes erreichen kann, über die gesamte Konusfläche des metallischen Halses und der keramischen Gelenkkugel eingeleitet wird, wodurch die Richtung der Krafteinleitung wesentlich vom Mittelpunkt der Gelenkkugel abweicht. Dies führt bei Dauerbelastung oftmals zum Versagen der Keramik, d.h. zur Rissbildung und in der Folge zum Bruch. Bereits kleinste Risse verursachen den gefürchteten Abrieb bei der Materialkombination Keramik-Metall, was letztendlich eine Erhöhung des Risikos einer vorzeitigen Revisionsoperation für den Patienten nach sich zieht.
Ein weiterer Nachteil besteht darin, dass wenn insbesondere bei Revisionsoperationen der Konuswinkel des Prothesenhalses unbekannt und/oder der Außenkonus des Prothesenhalses beschädigt ist, oftmals trotz eines festen Sitzes des Prothesenschaftes eine Extraktion letzteren mit all seinen nachteiligen Folgen für den Patienten durchgeführt werden muss.

### Aufgabenstellung

Bei diesem Stand der Technik liegt der Erfindung die Aufgabe zugrunde, ein Gelenkprothesen-System aus einer Metall-Keramik-Kombination bereitzustellen, das es ermöglicht, Winkelfehler des Konus am Prothesenhals bei gleichzeitiger Erhöhung der Prothesenlebensdauer sicher auszugleichen und die Gelenkkugel aus Keramik auf den Hals einer Prothese auch dann passgenau aufzusetzen, wenn die Konusabmessung des Prothesenschaftes nicht bekannt ist.

Diese Aufgabe wird durch ein Gelenkprothesen-System der eingangs genannten Gattung mit den Merkmalen des Anspruches 1 gelöst.

Vorteilhafte Ausgestaltungen des erfindungsgemäßen Gelenkprothesen-Systems sind den Unteransprüchen entnehmbar

Die erfindungsgemäße Lösung beruht auf der Erkenntnis, dass durch eine besondere Elastizität und eine spezielle Formgebung eines inneren und äußeren Wandabschnittes des Adapters die Krafteinleitung auf einen schmalen Flächenbereich am metallischen Außenkonus und die Krafteinleitung in den keramischen Innenkonus flächig auf einen dem schmalen Flächenbereich gegenüberliegenden lasttragenden Bereich innerhalb der größten Masseausdehnung der keramischen Gelenkkugel konzentriert ist.

Dies gelingt erfindungsgemäß durch einen Adapter, der als ein konischer Formkörper ausgebildet ist, dessen innerer Wandabschnitt eine zum Außenkonus des Halses hin konvex umlaufend geformte Aufwölbung zur gezielten Konzentration der Krafteinleitung auf den schmalen Flächenbereich des zum oberen Teil des Halses gehörenden Außenkonus des Schaftes, einen zum Innenkonus der Gelenkkugel hin kegelig ausgebildeten, dem inneren Wandabschnitt zugeordneten äußeren Wandabschnitt zur flächenhafte Anlage am lasttragenden Bereich des Innenkonus der Gelenkkugel und einen federnden Wandabschnitt aufweist, der sich unterhalb des lastragenden Bereichs am unteren Teil zunächst am Außenkonus zum Verhindern eines durch die konvex umlaufend geformte Aufwölbung erzeugten Kippmoments und im weiteren Verlauf elastisch gegen den Innenkonus der Gelenkkugel abstützt.

Dies ist mit dem außerordentlichen Vorteil verbunden, dass die keramische Gelenkkugel nur in dem Bereich beansprucht wird, der sehr nahe am virtuellen Mittelpunkt der Gelenkkugel liegt und dadurch in der Lage ist, Kräfte im gewissen Umfang aufzunehmen. Die Bereiche, die weiter von virtuellem Mittelpunkt der keramischen Gelenkkugelentfernt liegen, werden entlastet und müssen nur dem Kippmoment der Gelenkkugel entgegenwirken.

Von besonderem Vorteil ist weiterhin, wenn der Formkörper aus einem superelastischen Werkstoff, vorzugsweise einer biokompatiblen Formgedächtnislegierung, beispielsweise Nickel-Titan-Legierung, besteht, die eine ausgeprägte Superelastizität zeigt, wodurch der Adapter in Abhängigkeit des auf ihn wirkenden Spannungsniveaus eine Hysterese durchläuft, welche den Adapter wieder in seine Ausgangsform zurückbringt. Der erfindungsgemäße Adapter ist dadurch in der Lage seine bevorzugte Anlageposition am Hals der Prothese und am Innenkonus der Gelenkkugel beizubehalten, so dass sich die Krafteinleitung auf einem schmalen Bereich am Außenkonus des Halses und auf den lasttragenden Bereich am Innenkonus der keramischen Gelenkkugel entsprechend einstellt. Mit anderen Worten, der metallische Außenkonus wird mit hohen Belastungen beaufschlagt, wohingegen der keramische Innenkonus auf einer hinreichend großen Kontaktfläche geringfügig belastet wird, um die keramische Gelenkkugel nicht zu gefährden.

Es hat sich gezeigt, dass der Formkörper auch aus einer biokompatiblen Titan-Legierung bestehen kann.

In einer bevorzugten Ausführungsform des erfindungsgemäßen Gelenkprothesen-Systems weist der Formkörper in seiner Längsrichtung mindestens eine Aussparung auf, die dazu dient, einer unter Last induzierte Änderung im Habitus des Formkörpers entgegenzuwirken.

Es hat sich gezeigt, dass Aussparungen mit einer schlitzartigen oder v-Form dazu besonders geeignet sind. Vorteilhafterweise können auch mehrere Aussparungen übereinander in einer Flucht oder gegeneinander versetzt am Umfang angeordnet werden.

Nach einer bevorzugten besonderen Ausführungsform der Erfindung weisen die Formkörper für unterschiedlich lange Halsabschnitte des Schaftes entsprechend angepasste axiale Längen auf, um nach den operativen Gegebenheiten den Adapter mit geeigneter Länge auswählen zu können.

Gemäß einer weiteren bevorzugten Ausgestaltung der Erfindung weisen die Formkörper für unterschiedliche große Durchmesser von Halsabschnitten des Schaftes und für verschiedene Winkel des Außenkonus des Schaftes und des Innenkonus der Gelenkkugel die Formkörper entsprechend angepasste Durchmesser und Konenwinkel auf, so dass gewährleistet ist, dass der Chirurg den zum Einsatz geeigneten superelastischen Adapter entsprechend den tatsächlich vorhandenen Winkel- und Durchmesserabweichungen beim Patienten sicher auswählen kann.

In einer weiteren bevorzugten Ausführungsform der Erfindung bilden Formkörper mit unterschiedlich axialen Längen, Durchmessern und Winkeln Gruppen von Adaptern, denen jeweils Probeadapter zum Ermitteln eines passgenauen Sitzes von Schaft und Gelenkkugel zugeordnet sind.
Dies stellt sicher, dass der Chirurg den geeigneten Adapter schnell und problemlos während der Operation ermitteln kann.

Bei einer vorteilhaften Weiterbildung der Erfindung weisen die Gruppen von Adaptern eine entsprechende Durchmesserabstufung für Schaftdurchmesser aller handelsüblichen Prothesen auf. Die Abstufung gestattet es, mögliche Winkelfehler am Außenkonus des Halses von handelsüblichen Prothesen unterschiedlicher Hersteller und Abmessungen durch das elastische Verhalten des erfindungsgemäßen Adapters auszugleichen.

In einer besonders vorteilhaften Ausgestaltung der Erfindung ist zwischen der keramischen Gelenkkugel und dem erfindungsgemäßen Adapter ein weiterer Adapter vorgesehen, so dass es möglich wird, handelsübliche keramische Gelenkkugeln mit ihren dazugehörigen Adaptern einzusetzen und entsprechend zu kombinieren.

Weitere Vorteile und Einzelheiten ergeben sich aus der nachfolgenden Beschreibung unter Bezugnahme auf die beigefügten Zeichnungen.

### Ausführungsbeispiel

Die Erfindung soll nachstehend an einem Ausführungsbeispiel näher erläutert werden. Es zeigt
Fig. 1 einen Schnitt durch das Gelenkprothesen-System,
Fig. 2a und 2b vergrößerte Darstellungen des Adapters zwischen Innenkonus der keramischen Gelenkkugel und dem Außenkonus des Halses des metallischen Schaftes mit unterschiedlichen Konenwinkel gemäß Fig. 1,
Fig. 3 eine perspektivische Ansicht eines Adapters und
Fig. 4 ein Beispiel zu einer Gruppe zusammengefasster Probeadapter.

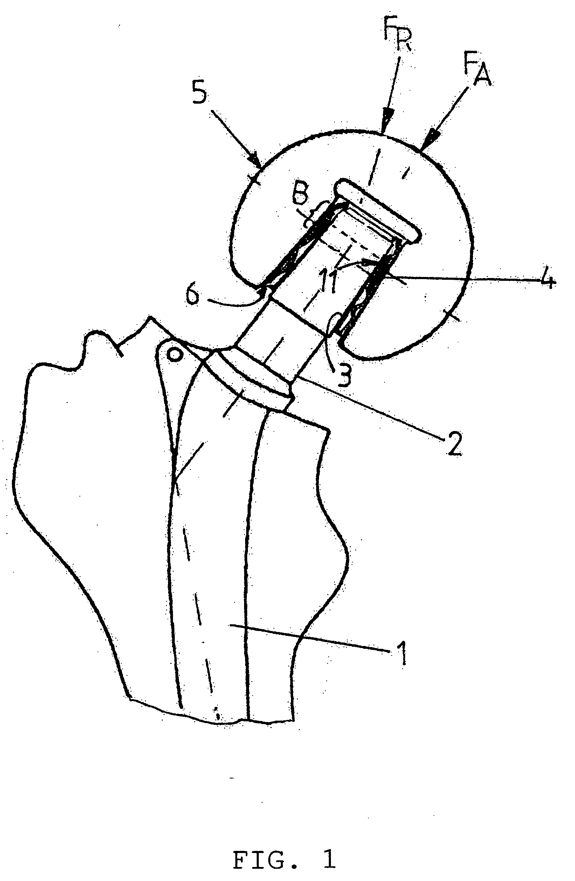

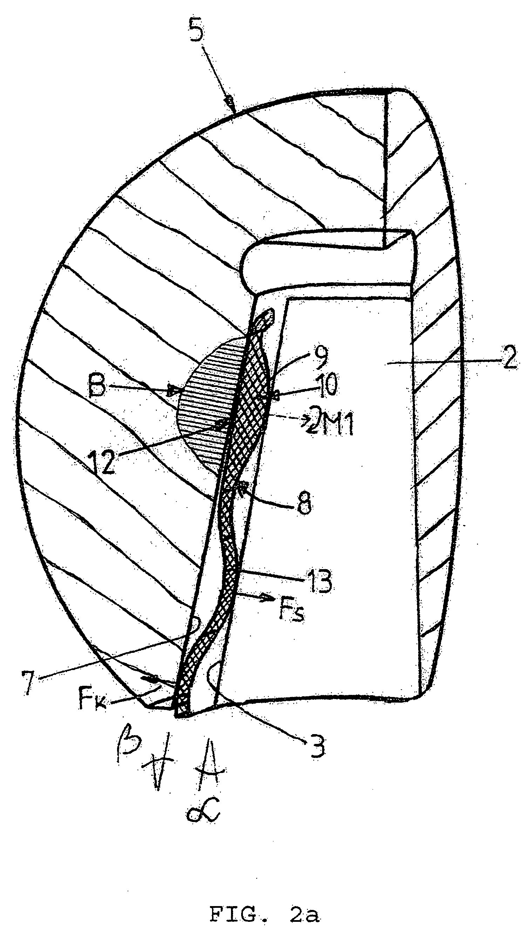

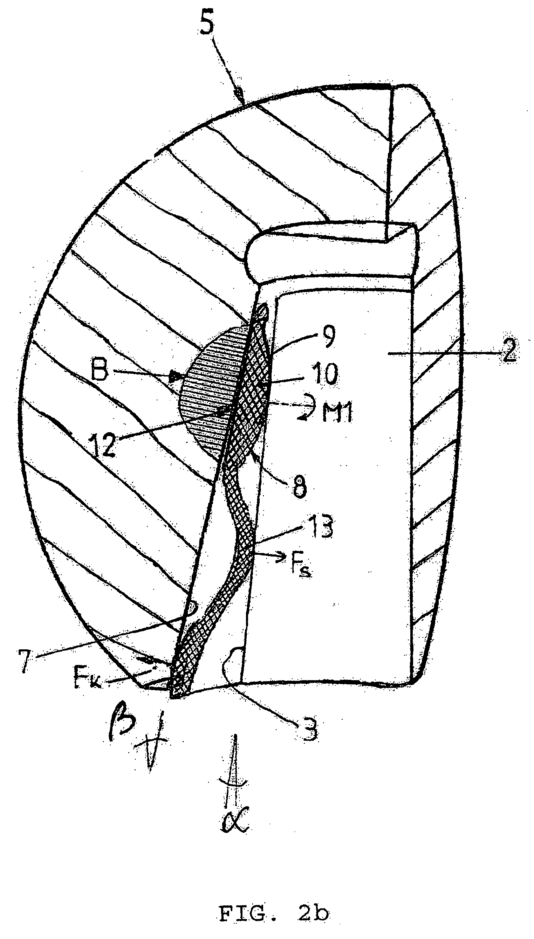

Die Fig. 1 zeigt einen Schnitt durch das erfindungsgemäße Gelenkprothesen-System. Der Schaft 1 der erfindungsgemäßen Gelenkprothese ist in den Markraum des Femur eingesetzt und an die anatomische Markraumsituation entsprechend angepasst. An den Schaft 1 schließt sich ein Hals 2 mit einem Außenkonus 3 an, auf dem ein Adapter 4 aufgesteckt -ist. Die keramische Gelenkkugel 5 ist mit einer Aufnahme 6 versehen, die als Innenkonus 7 (siehe Fig. 2) ausgebildet ist. Der Innenkonus 7, der Adapter 4 und Außenkonus 3 bilden eine Konus-Steckverbindung aus, die durch die beiden Durchmesser am jeweiligen Innen- bzw. Außenkonus sowie ihren dazugehörigen Konenwinkel α und β definiert sind. In der Fig. 1 sind schematisch die auf das Hüftgelenk wirkende resultierende physiologische Kraft F_{R} und die Axialkraft F_{A} angegeben, um die Kraftwirkungen auf die keramische Gelenkkugel 5 zu verdeutlichen. Die Gelenkkugel 5 ist dort am besten in der Lage, Lasten aufzunehmen, wo sie ihre größte Masseausdehnung in Bezug auf Ihren virtuellen Mittelpunkt besitzt (siehe Bereich B in Fig. 2).

Die Fig. 2a und 2b zeigen jeweils eine vergrößerte Darstellung des zwischen Innenkonus 7 der Gelenkkugel 5 und Außenkonus 3 des Halses angeordneten Adapters 4 (s.a. Fig. 1) bei unterschiedlichem Konuswinkel α des Außenkonus 3. Der Adapter 4 ist als ein hülsenförmiger Formkörper 8 ausgebildet und besteht aus einer biokompatiblen Formgedächtnislegierung, beispielsweise einer Nickel-Titan-Legierung wie Nitinol (Markenname der Nickel Titanium Naval Ordonance Laboratory) mit etwa 50 Gew.-% Nickel und Rest Titan. Der Konuswinkel β des Innenkonus 7 der Gelenkkugel 5 ist konstant. Der Adapter 4 besitzt somit sehr hohe superelastische Verformbarkeit, hohe Knickfestigkeit und ein ausgeprägtes Hystereseverhalten, wodurch dieser Werkstoff besonders für Klemmverbindungen geeignet ist.

Der Formkörper 8 des Adapters 4 weist einen inneren Wandabschnitt 9 auf, der zum Außenkonus 3 des Halses 2 des Schaftes 1 hin mit einer umlaufenden konvexen Aufwölbung 10 versehen ist, die sich in ihrem höchsten Punkt auf einem schmalen Flächenbereich 11 des zum oberen Teil des Halses 2 gehörenden Außenkonus 3 abstützt. Dies stellt sicher, dass die einwirkenden Kräfte auf der Metallseite der Klemmverbindung in den schmalen Flächenbereich konzentriert werden.
Dem inneren Wandabschnitt 9 ist ein äußerer Wandabschnitt 12 am Formkörper 8 zugeordnet, der entsprechend der Kontur des Innenkonus 7 der keramischen Gelenkkugel 5 kegelig ausgebildet ist und damit flächenhaft am lasttragenden Bereich B des Innenkonus 7 der Gelenkkugel 5 durch Verkippen zur Anlage kommt, so dass die auf die Gelenkkugel wirkenden Kräfte in den lasttragenden Bereich gleichmäßig verteilt werden. Dies sollen die Fig. 2a und 2b deutlich machen.
Der innere und äußere Wandabschnitt 9 bzw. 12 geht in einen federnden Wandabschnitt 13 über, der sich unterhalb des lasttragenden Bereichs B und der Aufwölbung 10 befindet und sich am metallischen Hals 2 des Außenkonus 3 mit einer Federkraft FS federnd abstützt, wobei der untere Teil des federnden Wandabschnitts 13 seinerseits auf den Innenkonus 7 der Gelenkkugel 5 elastisch anliegt und das Kippmoment M1 der Gelenkkugel 5 durch die Federkraft FK abfängt. Die federnden Eigenschaften des Wandabschnitts 13 kompensieren eventuell auftretende Durchmesseränderungen und/oder Winkeländerungen des metallischen Außenkonus 3 des Schaftes 2 der Prothese, wobei die elastische Abstützung sich so dimensionieren lässt, dass ein Bruch der Gelenkkugel 5 sicher ausgeschlossen werden kann.

Infolge der superelastischen Eigenschaften der Formgedächtnislegierung werden so vorhandene Winkel- und Durchmesserfehler zwischen Innen- und Außenkonus 3 bzw. 7 sicher ausgeglichen.

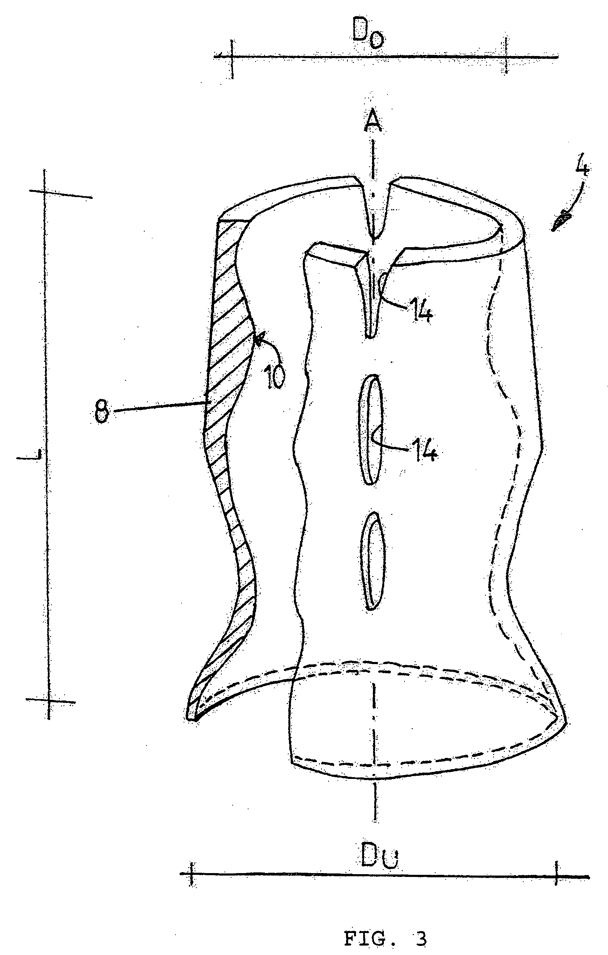

Die Fig. 3 zeigt den hülsenförmigen Formkörper 8 in perspektivischer Ansicht. In Längsachsenrichtung A des Formkörpers 3 besitzt der Formkörper 8 schlitzförmige Ausnehmungen 14, die eine elastische Änderung des Durchmessers des Adapters 4 zulassen. Der Formkörper 8 ist durch seine Konizität charakterisiert, d.h. der Veränderung des oberen Durchmessers DO auf den unteren Durchmesser DU über die Konushöhe L.
Der Innenkonus 7 der Aufnahme 6 der Gelenkkugel 5 und der Außenkonus 3 des Halses 2 des Schaftes der Prothese müssen aufeinander abgestimmt sein. So kann eine solche Schnittstelle aus entsprechend abgestuften Konenpaaren mit beispielsweise Durchmessern von 8, 10 oder 12 mm für den Innenkonus und von 10, 12 oder 14 mm für den Außenkonus gebildet sein. Der Konuswinkel α beträgt zwischen 4,5 und 7°.

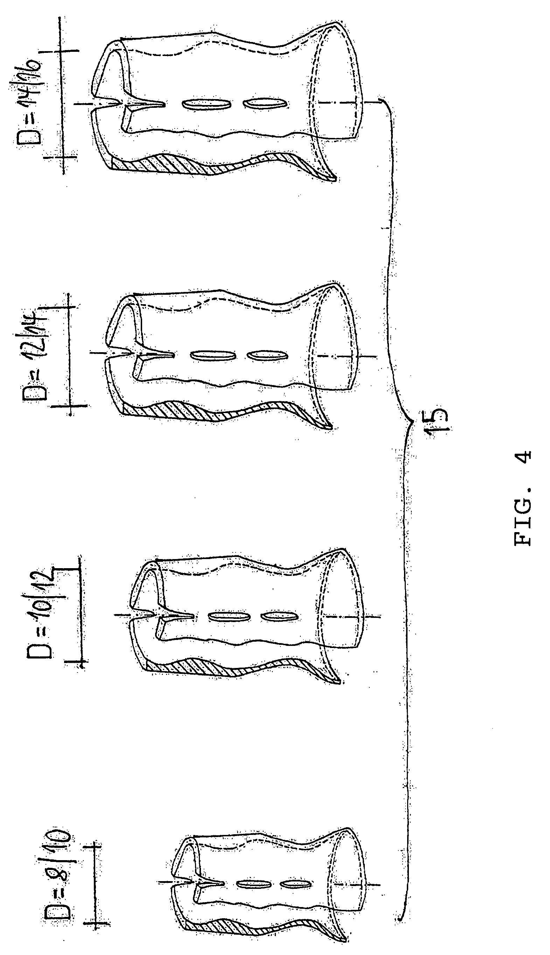

Die Fig. 4 zeigt beispielhaft eine Gruppe von Probeadaptern 15, deren Durchmesser entsprechend voneinander abgestuft sind.
Die Abstufung ist so gewählt, dass jeweils alle gängigen Längen, Durchmesser und Winkel der handelsüblichen Gelenkprothesen abdeckbar sind.

### Bezugszeichenliste

- Schaft: 1
- Hals: 2
- Außenkonus: 3
- Adapter: 4
- Gelenkkugel: 5
- Aufnahme in 5: 6
- Innenkonus von 6: 7
- Formkörper: 8
- Innerer Wandabschnitt von 8: 9
- Konvexe Aufwölbung: 10
- Flächenbereich der Krafteinleitung: 11
- Äußerer Wandabschnitt von 8: 12
- Federnder wandabschnitt: 13
- Ausnehmungen in 8: 14
- Probeadapter: 15
- Längsachse von 8: A
- Lasttragender Bereich von 5: B
- Oberer Durchmesser von 4: DO
- Unterer Durchmesser von 4: DU
- Axialkraft: F_{A}
- physiologische Kraft: F_{R}
- Federkraft auf Außenkonus 3: FS
- Federkraft auf Innenkonus 7: FK
- Konenwinkel: α, β

## Patentansprüche

1. Gelenkprothesen-System mit einem in den Femur implantierten metallischen Schaft (1) mit einem einen Außenkonus (3) aufweisenden Hals (2), einer Gelenkkugel (5) aus Keramik mit einer einen Innenkonus (7) umfassenden Aufnahme (6) für den Hals (2) und einem hülsenförmigen Adapter (4), der zwischen Schaft (1) und Gelenkkugel (5) klemmend angeordnet ist, wobei durch eine besondere Elastizität und eine spezielle Formgebung eines inneren und äußeren Wandabschnittes (9, 12) des Adapters (4) die Krafteinleitung auf einen schmalen Flächenbereich (11) am metallischen Außenkonus (3) und die Krafteinleitung in den keramischen Innenkonus (7) flächig auf einen dem schmalen Flächenbereich (11) gegenüberliegenden lasttragenden Bereich (B) innerhalb der größten Masseausdehnung der keramischen Gelenkkugel (5) konzentriert ist, **dadurch gekennze**i**chnet**, dass der Adapter (4) als ein konischer Formkörper (8) ausgebildet ist, dessen innerer Wandabschnitt (9) eine zum Außenkonus (3) des Halses (2) hin konvex umlaufend geformte Aufwölbung (10) zur gezielten Konzentration der Krafteinleitung auf den schmalen Flächenbereich (11) des zum oberen Teil des Halses (2) gehörenden Außenkonus (3) des Schaftes (2), einen zum Innenkonus (7) der Gelenkkugel (5) hin plan ausgebildeten, dem inneren Wandabschnitt (9) zugeordneten äußeren Wandabschnitt (12) zur flächenhafte Anlage am lasttragenden Bereich (B) des Innenkonus (7) der Gelenkkugel (5) und einen federnden Wandabschnitt (13) aufweist, der sich unterhalb des lastragenden Bereichs (B) zunächst am unteren Teil des Außenkonus zum Verhindern eines durch die konvex umlaufend geformte Aufwölbung (10) erzeugten Kippmoments (M1) und im weiteren Verlauf elastisch gegen den Innenkonus (7) der Gelenkkugel (5) abstützt.

2. Gelenkprothesen-System nach Anspruch 1, **dadurch gekennze**i**chnet**, dass der Adapter (4) aus einem superelastischen Werkstoff, vorzugsweise einer Nickel-Titan-Legierung, besteht.

3. Gelenkprothesen-System nach Anspruch 1, **dadurch gekennze**i**chnet**, dass der Adapter (4) aus einer Titan-Legierung besteht.

4. Gelenkprothesen-System nach Anspruch 1, **dadurch gekennze**i**chnet**, dass der Formkörper (8) in Richtung seiner Längsachse (A) mindestens eine Aussparung (14) aufweist.

5. Gelenkprothesen-System nach Anspruch 4, **dadurch gekennze**i**chnet**, dass die Aussparung (14) schlitzartig ausgebildet ist und mehrere Aussparungen übereinander in Flucht der Längsachse (A) angeordnet sind.

6. Gelenkprothesen-System nach Anspruch 1, **dadurch gekennze**i**chnet**, dass das Gelenkprothesen-System ein Set aus mehreren Adaptern (4) umfasst, die an unterschiedlich lange Halsabschnitte des Schaftes (2)entsprechend angepasste axiale Längen (L) aufweisen.

7. Gelenkprothesen-System nach Anspruch 1, **dadurch gekennze**i**chnet**, dass das Gelenkprothesen-System ein Set aus mehreren Adaptern (4) umfasst, die an unterschiedliche große Durchmesser von Halsabschnitten des Schaftes entsprechend angepasste Durchmesser aufweisen.

8. Gelenkprothesen-System nach Anspruch 1, **dadurch gekennze**i**chnet**, dass das Gelenkprothesen-System ein Set aus mehreren Adaptern (4) umfasst, die an verschiedene Winkel (α,β) des Außenkonus (3) des Schaftes (2) und des Innenkonus (7) der Gelenkkugel (5) entsprechend angepasste Konenwinkel aufweisen.

9. Gelenkprothesen-System nach einem der vorhergehenden Ansprüche, **dadurch gekennze**i**chnet**, dass Adapter (4) mit unterschiedlich axialen Längen (L), Durchmessern (DO,DU) und Winkeln (α) Gruppen von Adaptern bilden, denen jeweils Probeadapter (15) zum Ermitteln eines passgenauen Sitzes von Schaft (2) und Gelenkkugel (5) zugeordnet sind.

10. Gelenkprothesen-System nach Anspruch 9, **dadurch gekennze**i**chnet**, dass die Gruppen von Adaptern eine entsprechende Durchmesserabstufung für Schaftdurchmesser aller handelsüblichen Prothesen aufweisen.

11. Gelenkprothesen-System nach Anspruch 1, **dadurch gekennze**i**chnet**, dass zwischen dem Innenkonus (7) der Gelenkkugel (5) und dem Adapter (4) ein weiterer Adapter angeordnet ist.

## Claims

1. A joint prosthesis system with a metallic stem (1) which is implanted in the femur and has a neck (2) comprising an outer cone (3), a joint ball (5) of ceramic material with a receptacle (6) for the neck (2) comprising an inner cone (7), and a sleeve-type adapter (4), which is arranged clampingly between stem (1) and joint ball (5), the introduction of force into a narrow surface region (11) on the metallic outer cone (3) and the introduction of force into the ceramic inner cone (7) being concentrated on a load-bearing region (B) opposite the narrow surface region (11) within the greatest dimensional extent of the ceramic joint ball (5) due to a particular resilience and specific shaping of inner and outer wall portions (9, 12) of the adapter (4), **characterised in that** the adapter (4) takes the form of a conical moulding (8), the inner wall portion (9) of which comprises arching (10) shaped convexly encompassingly towards the outer cone (3) of the neck (2) for targeted concentration of the introduction of force onto the narrow surface region (11) of the outer cone (3) of the stem (2) belonging to the upper part of the neck (2), an outer wall portion (12) associated with the inner wall portion (9) and flat in shape towards the inner cone (7) of the joint ball (5) for bearing against the load-bearing region (B) of the inner cone (7) of the joint ball (5), and a resilient wall portion (13) which rests below the load-bearing region (B) initially against the lower part of the outer cone to prevent a tilting moment (M1) produced by the convexly encompassingly shaped arching (10) and further on resiliently against the inner cone (7) of the joint ball (5).

2. A joint prosthesis system according to claim 1, **characterised in that** the adapter (4) consists of a superelastic material, preferably a nickel-titanium alloy.

3. A joint prosthesis system according to claim 1, **characterised in that** the adapter (4) consists of a titanium alloy.

4. A joint prosthesis system according to claim 1, **characterised in that** the moulding (8) comprises at least one opening (14) in the direction of the longitudinal axis (A) thereof.

5. A joint prosthesis system according to claim 4, **characterised in that** the recess (14) is slit-shaped and a plurality of openings are arranged one above the other in alignment along the longitudinal axis (A).

6. A joint prosthesis system according to claim 1, **characterised in that** the joint prosthesis system comprises a set of multiple adapters (4), which have axial lengths (L) adapted in accordance with different length neck portions of the stem (2).

7. A joint prosthesis system according to claim 1, **characterised in that** the joint prosthesis system comprises a set of multiple adapters (4), which have diameters adapted in accordance with different diameters of neck portions of the stem.

8. A joint prosthesis system according to claim 1, **characterised in that** the joint prosthesis system comprises a set of multiple adapters (4), which have cone angles adapted in accordance with different angles (α, β) of the outer cone (3) of the stem (2) and of the inner cone (7) of the joint ball (5).

9. A joint prosthesis system according to one of the preceding claims, **characterised in that** adapters (4) of different axial lengths (L), diameters (DO, DU) and angles (α) form groups of adapters which are each associated with test adapters (15) for determining a perfect fit of stem (2) and joint ball (5).

10. A joint prosthesis system according to claim 9, **characterised in that** the groups of adapters comprise corresponding diameter grading for stem diameters of all conventional commercial prostheses.

11. A joint prosthesis system according to claim 1, **characterised in that** a further adapter is arranged between the inner cone (7) of the joint ball (5) and the adapter (4).

## Revendications

1. Système de prothèse d'articulation comprenant une tige métallique (1) implantée dans le fémur avec un col (2) présentant un cône externe (3), une rotule (5) en céramique avec un logement (6), comprenant un cône interne (7), destiné au col (2) et un adaptateur (4) en forme de manchon, qui est agencé entre la tige (1) et la rotule (5) par coincement, dans lequel, grâce à une élasticité particulière et à un façonnage spécifique d'une section de paroi (9) intérieure et d'une section de paroi (12) extérieure de l'adaptateur (4), l'application de force est concentrée sur un secteur superficiel étroit (11) du cône externe (3) métallique et l'application de force dans le cône interne (7) en céramique est concentrée en surface sur un secteur (B) porteur de charge faisant face au secteur superficiel étroit (11), à l'intérieur de la plus grande étendue de masse de la rotule (5) en céramique, **caractérisé en ce que** l'adaptateur (4) est réalisé en tant que corps moulé conique (8) dont la section de paroi (9) intérieure présente une courbure (10) formée de manière convexe circulaire par rapport au cône externe (3) du col (2) en vue d'une concentration ciblée de l'application de force sur le secteur superficiel étroit (11) du cône externe (3), appartenant à la partie supérieure du col (2), de la tige (2), une section de paroi (12) extérieure associée à la section de paroi (9) intérieure et réalisée de manière plane par rapport au cône interne (7) de la rotule (5) en vue d'une mise en place planaire au niveau du secteur porteur de charge (D) du cône interne (7) de la rotule (5), et une section de paroi (13) résiliente, qui s'appuie en dessous du secteur porteur de charge (B), d'abord au niveau de la partie inférieure du cône externe afin d'empêcher un couple de renversement (M1) produit par la courbe (10) formée de manière convexe circulaire et ensuite de manière élastique contre le cône interne (7) de la rotule (5).

2. Système de prothèse d'articulation selon la revendication 1, **caractérisé en ce que** l'adaptateur (4) est constitué d'un matériau superélastique, de manière préférée un alliage nickel-titane.

3. Système de prothèse d'articulation selon la revendication 1, **caractérisé en ce que** l'adaptateur est constitué d'un alliage de titane.

4. Système de prothèse d'articulation selon la revendication 1, **caractérisé en ce que** le corps moulé (8) présente au moins un évidement (14) en direction de son axe longitudinal (A).

5. Système de prothèse d'articulation selon la revendication 4, **caractérisé en ce que** l'évidement (14) est réalisé à la manière d'une fente et plusieurs évidements sont agencés de manière superposée dans l'alignement de l'axe longitudinal (A).

6. Système de prothèse d'articulation selon la revendication 1, **caractérisé en ce que** le système de prothèse d'articulation comprend un ensemble constitué de plusieurs adaptateurs (4) qui présentent des longueurs axiales (L) adaptées de manière correspondante au niveau de sections de col de différentes longueurs de la tige (2).

7. Système de prothèse d'articulation selon la revendication 1, **caractérisé en ce que** le système de prothèse d'articulation comprend un ensemble constitué de plusieurs adaptateurs (4) qui présentent des diamètres adaptés de manière correspondante au niveau de diamètres de différentes tailles de sections de col de la tige.

8. Système de prothèse d'articulation selon la revendication 1, **caractérisé en ce que** le système de prothèse d'articulation comprend un ensemble constitué de plusieurs adaptateurs (4) qui présentent des angles de cône adaptés de manière correspondante au niveau d'angles (α, β) différents du cône externe (3) de la tige (2) et du cône interne (7) de la rotule (5).

9. Système de prothèse d'articulation selon l'une quelconque des revendications précédentes, **caractérisé en ce que** des adaptateurs (4) de différentes longueurs axiales (L), diamètres (DO, DU) et angles (α) forment des groupes d'adaptateurs auxquels sont respectivement associés des adaptateurs d'essai (15) afin de déterminer un ajustement de la tige (2) et de la rotule (5) avec un repérage précis.

10. Système de prothèse d'articulation selon la revendication 9, **caractérisé en ce que** les groupes d'adaptateurs présentent un étagement de diamètres proportionné pour les diamètres de tige de toutes les prothèses du commerce.

11. Système de prothèse d'articulation selon la revendication 1, **caractérisé en ce qu'**un autre adaptateur est agencé entre le cône interne (7) de la rotule (5) et l'adaptateur (4).
